# EUROPÄISCHE PATENTSCHRIFT

(11) **EP 0 511 232 B1**
(45) Veröffentlichungstag und Bekanntmachung des Hinweises auf die Patenterteilung: **22.06.1994**
(21) Anmeldenummer: 91901727.7
(22) Anmeldetag: 10.01.1991
(51) Int. Cl.: A01N 1/02, A61L 2/16

(54) **VERFAHREN ZUR ENTFERNUNG VON VIREN AUS BIOLOGISCHEN FLÜSSIGKEITEN**
PROCESS FOR REMOVING VIRUSES FROM BIOLOGICAL FLUIDS
PROCEDE DE SUPPRESSION DE VIRUS DANS LES LIQUIDES BIOLOGIQUES

(30) Priorität: 17.01.1990 DE 4001099
(43) Veröffentlichungstag der Anmeldung: 04.11.1992
(73) Patentinhaber: OCTAPHARMA AG, 8750 Glarus (CH); SCHWINN, Horst, Dr., D-35039 Marburg (DE); PARADIES, Henrich Hasko, Prof. Dr., D-58636 Iserlohn (DE)
(72) Erfinder: SCHWINN, Horst, D-3550 Marburg (DE); PARADIES, Henrich, Hasko, D-5860 Iserlohn (DE)
(74) Vertreter: Werner, Hans-Karsten, Dr.Dipl.-Chem.
(86) Internationale Anmeldenummer: EP9100028
(87) Internationale Veröffentlichungsnummer: WO9110360

(56) Entgegenhaltungen:
- EP-A- 0 131 740
- EP-A- 0 321 835
- WO-A-88/01507
- WO-A-89/12390
- US-A- 4 769 318

## Beschreibung

Gegenstand der vorliegenden Erfindung ist ein Verfahren zur Entfernung von Viren, insbesondere von phosphorlipidumhüllten Viren aus biologischen Flüssigkeiten, insbesondere proteinhaltigen Flüssigkeiten durch Einbringen von Dialkoxyphosphorverbindungen.

Bisher im Stand der Technik verwendete Alkoxyphosphorverbindungen sind Substanzen wie Di- oder Trialkylphosphate, die unter anderem verwendet worden sind, um phosphorlipidumhüllte Viren zu inaktivieren, wobei man von der Annahme ausging, daß die Phosphorverbindungen die Membran dieser Viren zerstören. So beschreibt beispielsweise die EP-A-0131740 ein Verfahren zur Herstellung von proteinhaltigen Zubereitungen, die praktisch frei sind von phosphorlipidhaltigen Viren, bei dem die Zubereitungen ausreichend lange mit einer wirksamen Menge von Di- oder Trialkylphosphaten in Berührung gebracht werden. Dieses Verfahren ist auf die verschiedensten biologischen Flüssigkeiten anwendbar, insbesondere jedoch zur Desinfizierung von Blut- und Plasmapräparaten von Viren, in denen die Proteine nicht oder nur möglichst geringfügig denaturiert werden sollen. Gerade bei derartigen Verfahren ist es jedoch nötig, anschließend die Di- oder Trialkylphosphate wieder aus der Lösung zu entfernen, was mit erheblichem Aufwand und meist auch erheblichen Verlusten an Proteinen verbunden ist.

Zur Entfernung der bisher verwendeten Di- oder Trialkylphosphate wird im Stand der Technik mit Detergentien unter Anwendung eines schwer zu handhabenden Extraktionsverfahrens gearbeitet, um Detergenz und Di- oder Trialkylphosphat aus den Konzentraten zu eliminieren.

Andererseits ist es jedoch auch notwendig, eine möglichst vollständige Virusinaktivierung der Proteine zu erreichen, um das Risiko einer Virusinfektion bei der Behandlung mit Plasmaproteinen zu minimieren.

Die Erfindung hat sich daher die Aufgabe gestellt, mit dem Vorteil, im Verfahren zur Abtrennung von zur Inaktivierung von Viren in biologischen Flüssigkeiten verwendeten Dialhoxyphosphorverbindungen bereitzustellen, das mit geringem Aufwand arbeitet und deshalb in der Lage ist, größere Ausbeuteverluste zu vermeiden.

Es wurde jetzt überraschenderweise gefunden, daß diese Aufgabe dadurch gelöst werden kann, daß Dialkoxyphosphorverbindungen eingesetzt werden in Form von mechanisch abtrennbaren, trägergebundenen Dialkoxyphosphorverbin- dungen der allgemeinen Formel I

Tr-Spac(-O)ₙ-PO(O-Alk)₂ (I)

in der Tr einen inerten Träger, Spac einen Spacer, n die Zahlen 0 und 1 und Alk Alkylgruppen mit 1 bis 10 Kohlenstoffatomen bedeuten.

Die Anmelderin hat mit gleichem Einreichungstag eine Patentanmeldung mit der Anmeldungs-Nr. P 40 01 098.8-43 beim DPA hinterlegt, die trägergebundene Dialkylphosphorverbindungen und ihre Herstellung und Verwendung beschreibt.

Bei einer ausreichenden Beladung der Säulenmatrix mit den erfindungsgemäßen Dialkylphosphorverbindungen, und einer entsprechenden Kontaktzeit der wäßrigen Lösung, welche die Plasmaproteine enthält, ist es möglich, phosphorlipidhaltige Viren zu inaktivieren, so daß das Eluat ausschließlich die labilen Plasmaproteine enthält. Diese sind virusinaktiviert aber nicht denaturiert, wobei die Inaktivierung zumindest 4 bis 5 Zehnerpotenzen gegenüber dem Anfangstiter beträgt. Die Proteinaktivität im Eluat sowie der native Zustand dieser Proteine ist durch diese Behandlung nicht berührt und liegt bei über 80 %. Die entsprechende Verweildauer zur Inaktivierung der phosphorlipidhaltigen Viren kann durch die Fließgeschwindigkeit auf der Säule reguliert werden, so daß eine optimale Inaktivierung gewährleistet ist. Diese so präparierte Säulenmatrix ist außerdem hydrolyseresistent gegenüber hohen pH-Werten sowie gegenüber organischen Lösungsmitteln wie Ethanol, Methanol, DMSO, N,N-Dimethylformamid und N-Metylformamid sowie ionischen und nicht-ionischen Detergentien und Glykolen. Auf diese Art und Weise kann eine gepufferte wäßrige Lösung von Plasmaproteinen nach Passieren einer so hergestellten Matrix durch Säulenchromatographie phosphorlipidhaltige Viren inaktivieren. Solche Lösungen von Plasmaproteinen können Gerinnungsproteine, wie Faktor VIII aber auch Lösungen anderer Proteine, Nukleinsäuren und zusammengesetzter Proteinkomplexe (z.B. Plasmaproteine, Gerinnungsfaktoren Albumin, Phosphofructokinase, H⁺-ATPasen, cAMP-Proteinkinasen) und Lösungen, die ganze Zellen verschiedener Herkunft enthalten (z. B. Zellkulturen, die für in vitro-Studien verwendet werden), sein.

Nach dem erfindungsgemäßen Verfahren werden fast alle phosphorlipidhaltigen Viren, insbesondere die Hepatitis- und HIV-Viren, inaktiviert. Die Inaktivierung wurde auch mit der Herpes-Virengruppe sowie der Influenza-A-Viren-Gruppe nach dem Plaque-Assay gemäß K. Tobita, A. Suginere, C. Enamote und F. Fusiyama, Med. Microbiol. Immuniol. 162, 9 (1975) an Nierenepithelien (Hund, MDCK) in Gegenwart von Trypsin durchgeführt. Die Inaktivierung wurde im Verhältnis der unbehandelten zur behandelten Probe gemessen, so daß nach dem Verdünnungsprinzip bei einer Verdünnung von 10⁴ bis 10⁵ eine virale Aktivität noch nachweisbar war, und somit die Inaktivierung der phosphorlipidhaltigen Viren experimentell meßbar ist.

Das erfindungsgemäße Verfahren ist auch für Nichtblutkomponenten wie Nukleinsäuren (DNA, RNA, cDNA, hnmRNA) aus Pro- und Eukaryonten mit Kohlenhydrat bzw. Polysaccharidbestandteilen geeignet.

Als Träger kommen insbesondere anorganische oder organische Festkörper oder Gele in Frage, welche durch kovalente Bindungen mit einem Spacer verknüpft werden können. Als anorganische Träger kommen beispielsweise Gläser, Kieselsäuren, Aluminiumhydroxide und sonstige inerte anorganische Träger in Frage, wie sie auch für trägergebundene Enzyme zur Anwendung kommen. Als organische Träger kommen wiederum alle üblicherweise eingesetzten inerten Träger in Frage insbesondere vernetzte und unvernetzte polymere Kohlehydrate, wie Cellulosen, Dextrane, Agarosen, Polyacrylate und Copolymerisate. Besonders bevorzugt sind Sepharose^{R}, Sephacel, Fractogel^{R} und Sephadex^{R}, und deren bereits aktivierte Abkömmlinge, die bereits einen Spacer enthalten, wie z.B. Brom-acetamido-alkyl-derivate, CNBr-Sepharose oder O-Bromacetyl-N-Hydroxy-Succinimid.

Als Spacer werden im allgemeinen geradkettige Alkylgruppen gewählt mit 2 bis 8 Kohlenstoffatomen, die an beiden Enden kovalente Bindungen eingehen können. Besonders bevorzugt werden Spacer mit 6 Kohlenstoffatomen, darüber hinaus können auch alle übrigen bekannten Spacer eingesetzt werden, die insbesondere für trägergebundene Enzyme entwickelt worden sind.

Die Alkylgruppen der Phosphorverbindungen können je nach Verwendungszweck zwischen 1 und 10 Kohlenstoffatomen aufweisen. Bevorzugt werden solche mit 3 bis 6 Kohlenstoffatomen. Für die Entfernung von Viren mit einer Phospholipidhülle, wie Hepatitis B hat sich insbesondere eine Kettenlänge von 4 Kohlenstoffatome besonders bewährt.

Die weiteren Untersuchungen dieser neuen trägergebundenen Dialkoxyphosphorverbindungen hat ergeben, daß durch Variationen des Trägers, des Spacers und der Länge der Alkylgruppen durchaus unterschiedliche Ergebnisse erzielt werden können. So ist einerseits darauf zu achten, daß der Träger nicht nur gegenüber den Proteinen inert ist, sondern auch nicht dazu neigt, diese unverhältnismäßig hoch zu adsorbieren, wodurch es zu unerwünschten Verlusten kommen kann. Das gleiche gilt für die Länge des Spacers. Zur Auf- und Abtrennung von lipophilen Substanzen können vielfach schon sehr kurze Spacer ausreichen. Insbesondere um die Phospholipidhülle von Hepatitis B Viren zu zerstören, ist eine Mindestlänge des Spacers erforderlich. Zu lange Spacer wiederum führen zu geringerer Effizienz der trägergebundenen Dialkoxyphosphorverbindungen. Für diesen Zweck haben sich daher Spacer-Längen von 6 Kohlenstoffatomen als optimal erwiesen.

Die kovalente Kopplung des Phosphoratoms mit dem Spacer kann unter Zuhilfenahme übliche Kupplungsreagentien erfolgen, wie beispielsweise Oxiranen, Epoxiden, Carbodiimiden. Während bei der Verwendung von Di- und Trialkylphosphaten nach dem Stand der Technik bisher nur Ester der Phosphorsäure zur Anwendung gekommen sind, können erfindungsgemäß auch die Alkylphosphonate über einen Spacer an Träger gebunden werden. Diese trägergebundenen Alkylphosphonate weisen ebenfalls hervorragende Eigenschaften auf bezüglich der Auf- und Abtrennung von lipophilen Substanzen sowie der Inaktivierung und der Entfernung von Viren mit einer Phospholipidhülle.

Es hat sich weiterhin gezeigt, daß es mit Hilfe der trägergebundenen Dialkoxyphosphorverbindungen möglich ist, aus biologischen Flüssigkeiten mit hoher Effizienz Viren zu entfernen, ohne die gewünschten Proteine zu denaturieren. Bei Auswahl geeigneter Träger und Spacer ist es sogar möglich, die Ausbeuteverluste an gewünschten Proteinen extrem gering zu halten. Weiterhin wurde gefunden, daß es mit Hilfe der erfindungsgemäßen trägergebundenen Dialkoxyphosphorverbindungen möglich ist, lipophile Substanzen von hydrophilen Substanzen abzutrennen und sogar Substanzen mit gradueller Abstufung der Lipophilität chromatographisch aufzutrennen, so daß mit ihnen neue Formen der Affinitätschromatographie möglich sind.

In den nachfolgenden Beispielen sind einige bevorzugte Ausführungsformen des erfindungsgemäßen Verfahrens zur Inaktivierung von Viren beschrieben:

### Beispiel 1

5 l Fractogel, beladem mit chemisch fixiertem Dibutylphosphat (50 - 1000 »mol/ml) werden in 0,15 mol/l NaCl suspendiert und in eine Chromatographiesäule gefüllt, so daß die Höhe des Gelbettes etwa gleich groß ist wie der Durchmesser der Säule.

5 l Human-Citrat-Plasma werden filtriert (Porengröße 1 »m) und mit Herpes-Simplex-Viren (HSV) versetzt.

Das Plasma passiert anschließend bei Raumtemperatur das Gelbett im Verlauf von 6 Stunden.

Danach ist kein virulentes HSV mehr nachweisbar.

### Beispiel 2

Eine Chromatographiesäule mit Dibutylphosphat-beladenem Fractogel wird vorbereitet wie unter 1 beschrieben.

50 l einer Faktor VIII-haltigen Lösung, die 120 »mol/l NaCl, 10 mmol/l Na₃-Citrat, 1 mmol/l CaCl₂ und 120 mmol/l Glycin enthält, werden filtriert (1 »m) und wie unter 1 mit HSV versetzt. Die Faktor VIII-Lösung passiert das Gelbett im Verlaufe von 12 Stunden bei Raumtemperatur.

Danach ist kein virulentes HSV mehr nachweisbar.

Die im l-Phasentest bestimmte Faktor VIII-Aktivität wird durch diese Behandlung nicht beeinträchtigt.

### Beispiel 3

5 l Fractogel, an dessen Oberfläche Dibutylphosphonat über Spacer chemisch fixiert wurde (50 - 1000 »mol/ml werden in einer temperierbaren Chromatographie-Säule vorbereitet wie unter 1 beschrieben. Das Gelbett wir mittels eines Ultrathermostaten auf + 30°C gehalten.

5 l Human-Citrat-Plasma werden filtriert (Porengröße 1 »m) und mit Polioviren versetzt.

Das Plasma passiert anschließend das Gelbett im Verlaufe von 6 Std. bei + 30°C.

Danach ist kein virulentes Polio-Virus mehr nachweisbar.

### Beispiel 4

5 l Dibutylphosphat-beladenes Fractogel werden vorbereitet wie unter 1.

50 l Human-Citrat Plasma werden filtriert (Porengröße 1 »m) und mit HSV versetzt.

Das Plasma passiert anschließend das Gelbett im Verlaufe von 24 Std. bei Raumtemperatur.

Danach ist kein virulentes HSV mehr nachweisbar.

### Bezugsbeispiel 1

21,0 g Di-n-butylphosphat werden in 100 ml 1,2-Dimethoxyethan bei 4°C gelöst. Unter stetigem Rühren werden 18,0 g N-ethyl-N′-(3-dimethyl-aminopropyl) carbodiimid, das in 75 bis 100 ml Wasser gelöst ist, tropfenweise zu der Lösung gegeben, bis eine Konzentration von 0,1 mol/l Carbodiimid erreicht ist. Der pH-Wert wird auf pH 5,1 bis 5,5 konstant gehalten. Die milchige Suspension wird mit 1,4-Dioxan (10 % w/w) versetzt, um die Löslichkeit von Di-n-butylphosphat zu erhöhen.

Es empfiehlt sich, das Gel, z.B. Fractogel, Agarose oder Sephadex unter kräftigem Rühren, vorzugsweise in einem Volumenverhältnis von Flüssigkeit (Suspension) zu Gel wie 2 : 1 (4°C, pH 5,5 - 6,0, maximal 6,0) mit Thionylchlorid in Pyridin (Gewichtsverhältnis 1 : 1) zu versetzen, um den pH-Wert während der Kupplungsreaktion möglichst konstant auf pH = 6,0 zu halten. Die Lösung von Di-n-butylphosphat und N-ethyl-N′-(3-dimethyl-aminopropyl) carbodiimid wird dann unter stetem Rühren tropfenweise zur Suspension zugegeben. Der beobachtbare pH-Abfall wird in den ersten 30 Minuten durch Pyridin/S0Cl₂ korrigiert (pH = 6,0, 4°C). Die Reaktion ist nach vier Stunden bei 4°C beendet.

### Bezugsbeispiel 2

Um aktivierte Sepharose 6 B mit einem hydrophoben Spacerarm an (CH₂)₄ herzustellen, verfährt man wie unter 1 beschrieben, läßt jedoch zuerst das Gel mit z.B. 1, 4 bis (2,3-epoxypropyloxy) butan, bei 20°C mit einem 10 %igen (w/w) Überschuß des Epoxides reagieren. Die Aufarbeitung gestaltet sich ebenso wie unter 1 ausgeführt.

Im Unterschied zum mehr hydrophoben Spacerarm liegt in diesem Fall ein Gemisch von hydrophilen in hydrophoben Bereichen innerhalb des Spacerarms vor:

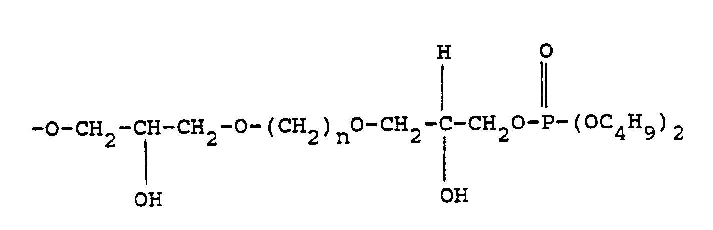

Die Verlängerung der (CH₂)ₙ-Gruppe kann u.a. durch Kondensation mit n-Dialkyl-diolen geschehen, die am Ende eine Epoxy-Gruppe enthalten. Die Beladungsdichte liegt bei einer Kettenlänge von n = 6 bei 120 »mol/g geschwollenem Gel im Fall von Sephadex G 100, oder 25 bis 30 »mol/g geschwollenem Gel bei Sepharose 6 B und 15 bis 18 »mol/g geschwollenem Gel bei Fractogel.

Eine Erhöhung der Beladung um das fünffache, bis zu 150 »mol/g geschwollenem Gel kann durch Hinzunahme des Kupplungsreagenzes erreicht werden.

Das Gel wird in einer Bowl-Zentrifuge oder in einer RC5B (Dupont) bei 2.000 UpM abzentrifugiert, und der Überstand verworfen. Der Rückstand wird mehrmals mit destilliertem Wasser gewaschen, abgenutscht und anschließend lyophilisiert.

Die Beladungsdichte des Gels mit dem Ligand einschließlich der Di-n-butylphosphat-Gruppe wurde durch Titration der o-Phosphorsäure nach Hydrolyse mit konzentrierter Salzsäure bestimmt. Alternativ dazu ist auch eine direkte Bestimmung durch HPLC-Chromatographie (Bondepak I 75, isotaktisch) des mit Di-n-butylphosphat beladenen Spacerarms, z.B. Tr-Spac(-O)ₙPO(OC₄H₉)₂ möglich.

Die Beladungsdichte liegt bei Sephadex G 100 oder Agarose-Gelen in der Größenordnung von 25 »mol/g hydratisiertem Gel oder 25»mol/g hydratisiertem Gel im Falle von Agarose A 1,5 m. Fractogel-Präparationen haben im Durchschnitt eine Beladungsdichte von 25 bis 30 »mol/g geschwollenem Gel.

### Bezugsbeispiel 3

### Herstellung von Di-n-butylphosphorsäurechlorid ((RO)₂P(O)Cl)

168 g Phosphoroxychlorid und 175 g n-Butanol (p.a) werden in einem 1 Liter Rundkolben mit Rückflußkühler und Thermometer gemischt und mittels eines Heizpilzes auf 130 bis 140°C erwärmt. Die entstehende Salzsäure wird mit NaOH aufgefangen. Die Reaktion ist nach 1 1/2 bis 2 Stunden beendet. Der Kolbeninhalt wird im Vakuum bei 0,8 bis 1,0 T fraktioniert und die entsprechende Fraktion bei Kp 95 bis 97°C und 1,5 T aufgefangen und redestilliert. Kp : 93 - 95°C / 1,5 T. Die Ausbeute beträgt ca. 190 g entsprechend 80 %.

Erwähnenswert ist, daß durch entsprechende Variation der Molverhältnisse auch das n-Butylphosphorsäure-dichlorid (Kp : 130°C, 760 T) erhalten werden kann.

Die CNBr aktivierte Agarose 2 B wird wie unter 2 beschrieben mit einem entsprechenden Spacerarm, z.B. mit 1,4-(2,3-epoxybutoxy)-Butan versehen und anschließend in 1 Liter Pyridin unter stetem Rühren die molare Menge von Di-n-butylphosphorsäurechlorid gelöst und 22,6 g Dicyclohexylcarbodiimid hinzugesetzt werden. Nach 60 Stunden bei 35°C wird vom Dicyclohexylharnstoff abfiltriert, zunächst mit reichlich Wasser, dann mit reichlich Ether gewaschen und anschließend mit 2 Liter 0,01 M Kalium-Phosphat-Pufferlösung vom pH 7,5 und 20°C gewaschen. Das so hergestellte Gel bzw. die Gelmatrix wird in 0,001 M Kalium-Phosphat-Pufferlösung bei pH 7,5 und 4°C in Gegenwart von Natriumazid aufbewahrt.

### Bezugsbeispiel 4

### Herstellung von Di-n-butylphosphonat und Di-n-butylphosphorsäurechlorid

Zu einer eisgekühlten Lösung von 15,0 g Phosphortrichlorid in 100 ml Benzol (Toluol) wird unter stetem Rühren innerhalb von 3 Stunden eine Mischung von 24,5 g Dimethylanilin und 22 g n-Butylakohol zugetropft. Unter Fortsetzen des Rührens werden nach weiteren 20 Minuten 12 g n-Butylalkohol (p.a.) in derselben Weise zugetropft. Die Reaktionsmischung läßt man über Nacht bei 20°C stehen, fügt 75 ml Wasser hinzu und wäscht die Benzol-(Toluol-) Phase im Scheidetrichter mehrmals mit 50 ml H₂O, dann mit 5 M Ammoniak und anschließend mit 500 ml Wasser aus. Nach dem Trocknen über wasserfreiem Natriumsulfat bzw. Molekularsieben wird das Benzol (Toluol) im Vakuum abgezogen. Das im Rückstand verbliebene Öl wird im Hochvakuum bei 10⁻² T und 70°C unter Stickstoffatmosphäre destilliert. Zur Destillation wird das Produkt zunächst mit Ammoniakgas gesättigt und in Gegenwart von 1 % N-methyl-morpholin destilliert. Das Di-n-butylphosphonat destilliert bei 78°C und 10⁻¹ T über. 15 g Di-n-butyl-phosphonat werden in 100 ml Tetrachlorkohlenstoff gelöst und bei -20°C langsam eine 1,0N Lösung von Chlor in Tetrachlorkohlenstoff hinzugegeben. Es wird unter stetem Rühren bei -20°C trockenes Stickstoffgas eingeleitet bis keine Salzsäure mehr entweicht. Nach Verdampfen des Tetrachlorkohlenstoffs bei 20°C destilliert das Säurechlorid als hellgelbe Flüssigkeit bei 0,5 T über. Es wird sofort in die Kopplungsreaktion eingesetzt.

2,5 g Di-n-butylphosphorsäurechlorid werden in 50 ml Pyridin (90 %) unter stetem Rühren bei 40°C in Gegenwart von 6-Chlorhexanol und 10 ml Tri-butylamin gelöst. Unter stetem Rühren und Herabsetzung der Temperatur auf 20°C wird 1 g Gel (Fractogel, Sepharose 6 B) eingerührt, und der Brei 60 Stunden bei 20°C stehen gelassen. Danach wird das Gel auf einer Nutsche mit mindestens 1 Liter Wasser gewaschen, danach mit 2 Liter 0,001 M Kalium-Phosphat-Pufferlösung bei pH 7,5 gespült. Das fertige Gel mit der Phosphorsäure-Matrix wird in Gegenwart von Natriumazid in 0,001 M Kalium-Phosphat-Pufferlösung bei 4°C aufbewahrt.

### Bezugsbeispiel 5

### 1-Chloroctyl-di-n-butylphosphonat

Eine Mischung von 25 g 1,8-Chloroctan und 25,5 g Tri-n--butylphosphit werden für 6 Stunden auf 100°C erhitzt. Anschließend wird bei 150 bis 160°C und 10 T destilliert und das übergehende 1-Chloroctyl-di-n-butylphosphonat in 70 %iger Ausbeute aufgefangen.

Der Ester wird in 25 ml Pyridin (90 %) in Gegenwart von 1 g Fractogel (Agarose 6 B) bei 20°C unter stetem Rühren an das Gel gekoppelt. Die Mischung wird 60 Stunden stehengelassen und anschließend auf der Nutsche mit 1 Liter Wasser, dann mit 2 Litern 0,01 M Di-Kaliumhydrogenphosphat bei pH 7,5 gewaschen. Das Gel wird in 0,001 M Di-Kaliumhydrogenphosphat bei pH 7,5 in Gegenwart von Natriumazid bei 4°C aufbewahrt.

## Patentansprüche

1. Verfahren zur Entfernung von Viren, insbesondere von phosphorlipidumhüllten Viren, aus biologischen Flüssigkeiten, insbesondere proteinhaltigen Flüssigkeiten durch Einbringen von Dialkoxyphosphorverbindungen, dadurch gekennzeichnet, daß die Dialkoxyphosphorverbindungen eingesetzt werden in Form von mechanisch abtrennbaren trägergebundenen Dialkoxyphosphorverbindungen der allgemeinen Formel I
Tr-Spac(-O)ₙPO(OAlk)₂ (I),
in der Tr einen inerten Träger, Spac einen Spacer, n die Zahlen 0 und 1 und Alk Alkylgruppen mit 1 bis 10 Kohlenstoffatomen bedeuten.

## Claims

1. A process for removing viruses, especially phospholipid-enveloped viruses, from biological fluids, in particular, protein-containing fluids, by incorporating dialkoxyphosphorus compounds, characterized in that the dialkoxyphosphorus compounds are employed in the form of mechanically separable, substrate-bonded dialkoxyphosphorus compounds of general formula I
Tr-Spac(-O)ₙ-PO(O-Alk)₂ (I)
wherein Tr represents an inert substrate, Spac represents a spacer, n represents the numbers 0 and 1, and Alk represents alkyl groups having from 1 to 10 carbon atoms.

## Revendications

1. Procédé d'élimination de virus - et en particulier de virus à enveloppe phospholipidique - de liquides biologiques, et en particulier de liquides contenant des protéines, par l'adjonction de composés de dialcoxyphosphore, caractérisé en ce que les composés de dialcoxyphosphore sont utilisés sous la forme de composés de dialcoxyphosphore liés à un support et mécaniquement séparables ayant la formule générale I
Tr-Spac(-O)nPO(OAlk)2 (I),
où Tr désigne un support inerte, Spac, un espaceur, n, les chiffres 0 et 1, et Alk, des groupes alkyles avec 1 à 10 atomes de carbone.
